# FASCICULE DE BREVET EUROPEEN

(11) **EP 0 749 748 B1**
(45) Date de publication et mention de la délivrance du brevet: **21.05.1997**
(21) Numéro de dépôt: 96401216.5
(22) Date de dépôt: 06.06.1996
(51) Int. Cl.: A61K 7/13

(54) **Compositions pour la teinture des fibres kératiniques comprenant un ortho-diamino pyrazole et un sel de manganèse, procédé de teinture mettant en oeuvre ces compositions**
Haarfärbemittel enthaltend ein ortho-Diaminopyrazol und ein Mangansalz und Färbeverfahren mit demselben Mittel
Compositions for dyeing keratinous fibers comprising ortho-diamino pyrazole and a manganese salt and dyeing process using such a composition

(30) Priorité: 21.06.1995 FR 9507433
(43) Date de publication de la demande: 27.12.1996
(73) Titulaire: L'OREAL, 75008 Paris (FR)
(72) Inventeur: Samain, Henri, 91570 Bievres (FR)
(74) Mandataire: Andral, Christophe André Louis

(56) Documents cités:
- EP-A- 0 375 977
- DE-A- 4 234 887
- FR-A- 2 090 272

## Description

L'invention a pour objet de nouvelles compositions pour la teinture des fibres kératiniques et en particulier des fibres kératiniques humaines telles que les cheveux, comprenant, en mélange ou de manière séparée, un ortho-diamino pyrazole et un sel de manganèse, ainsi qu'un procédé de teinture mettant en oeuvre ces compositions, la révélation de la coloration étant réalisée sans autre agent oxydant que l'oxygène de l'air.

Il est connu de teindre les fibres kératiniques et en particulier les cheveux humains avec des compositions tinctoriales contenant des précurseurs de colorant d'oxydation appropriés, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des agents oxydants convenables, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Les colorants d'oxydation (bases d'oxydation et coupleurs), utilisés pour la coloration permanente des fibres kératiniques sont généralement révélés à l'aide d'agents oxydants tels que l'eau oxygénée. Ce type de révélation permet certes d'obtenir des colorations puissantes, mais il n'est pas sans conséquence vis à vis des fibres kératiniques qui subissent inévitablement une décoloration accompagnée en outre d'une dégradation de la fibre.

Il a déjà été proposé, notamment dans la demande de brevet EP-A-375 977, d'utiliser certains dérivés d'ortho-diamino pyrazoles, à savoir plus précisément des dérivés de 3,4- ou de 4,5-diamino pyrazoles tels que par exemple le 4,5-diamino pyrazole, pour la teinture d'oxydation des fibres kératiniques dans des nuances rouges. Toutefois, le procédé de teinture décrit dans cette demande de brevet met en oeuvre un agent oxydant tel que par exemple l'eau oxygénée, de sorte qu'une coloration puissante ne peut être atteinte sans que la fibre kératinique ne soit altérée et décolorée.

Certes, il est connu que certains diamino pyrazoles, de structure très particulière, tels que ceux décrits dans les demandes de brevets DE-A-4 234 886 et DE-A-4 234 887 peuvent être révélés sans agent oxydant autre que l'oxygène de l'air, conduisant ainsi à des colorations n'altérant pas les fibres kératiniques.

Toutefois, dans le cas particulier des ortho-diamino pyrazoles qui ne peuvent pas être révélés avec le seul oxygène de l'air, comme dans celui, plus général, de la plupart des autres bases d'oxydation telles que par exemple l'ortho-diamino benzène, il n'est malheureusement pas possible d'obtenir une coloration satisfaisante sur cheveux lorsqu'on met en oeuvre un procédé de teinture ne faisant appel qu'à l'oxygène de l'air comme agent de révélation de la coloration.

Or, la demanderesse vient maintenant de découvrir, de façon totalement inattendue et surprenante, que les sels de manganèse permettent, et ceci sans avoir à mettre en oeuvre un agent oxydant autre que l'oxygène de l'air, de révéler de façon efficace les ortho-diamino pyrazoles qui ne peuvent habituellement être révélés à l'air.

Cette découverte est à la base de la présente invention.

La présente invention a donc pour objet une composition pour la teinture des fibres kératiniques et en particulier des fibres kératiniques humaines telles que les cheveux, caractérisée par le fait qu'elle comprend, dans un milieu approprié pour la teinture :
- au moins un ortho-diamino pyrazole et / ou au moins l'un de ses sels d'addition avec un acide, incapable d'être révélé efficacement par le seul oxygène de l'air,
- et au moins un sel de manganèse.

Dans les compositions selon l'invention, l'ortho-diamino pyrazole et le sel de manganèse peuvent se présenter sous la forme d'un mélange ou, au contraire, être conditionnés sous forme séparée.

La composition tinctoriale conforme à l'invention n'a pas besoin d'être mélangée au moment de l'emploi avec un agent oxydant classique tel que l'eau oxygénée ; elle conduit en outre à des colorations rapides, puissantes et tenaces, sans entraîner une décoloration ou une altération des fibres kératiniques.

Par ortho-diamino pyrazole incapable d'être révélé efficacement par le seul oxygène de l'air, on entend tout ortho-diamino pyrazole ne conduisant à aucune coloration satisfaisante des fibres kératiniques lorsqu'il est révélé uniquement par l'oxygène de l'air.

Les ortho-diamino pyrazoles incapables d'être révélés efficacement par le seul oxygène de l'air sont ainsi de préférence choisis parmi les composés de formule (I) suivante, et leurs sels d'addition avec un acide : 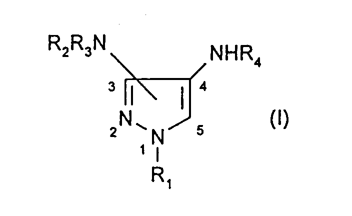 dans laquelle :
- R₁, R₂ et R₄, identiques ou différents, représentent un atome d'hydrogène, un radical alkyle en C₁-C₄, monohydroxyalkyle en C₂-C₄, benzyle ou phényle,
- R₃ représente un atome d'hydrogène, un radical alkyle en C₁-C₄ ou monohydroxyalkyle en C₂-C₄,
le groupement amino NR₂R₃ occupant soit la position 3, soit la position 5.

Parmi les composés de formule (I) ci-dessus, on peut plus particulièrement citer le 4,5-diamino pyrazole, le 4,5-diamino 1-méthyl pyrazole, le 1-benzyl 4,5-diamino pyrazole, le 3,4-diamino pyrazole, et leurs sels d'addition avec un acide.

Le 4,5-diamino pyrazole et ses sels d'addition avec un acide sont particulièrement préférés.

Les sels de manganèse pouvant être utilisés dans la composition tinctoriale conforme à l'invention n'ont pas d'activité oxydante propre et dans ces sels, le manganèse présente de préférence un degré d'oxydation égal à 2 ou à 3. On peut bien entendu utiliser un ou plusieurs sels de manganèse.

Ils sont de préférence choisis parmi le diacétate de manganèse et ses hydrates tels que par exemple le diacétate de manganèse tétrahydrate, le dichlorure de manganèse et ses hydrates, les sulfates de manganèse, les carbonates de manganèse, les dihydrogénocarbonates de manganèse, l'acétylacétonate de manganèse, le triacétate de manganèse et ses hydrates et le trichlorure de manganèse.

Le diacétate de manganèse tétrahydrate est particulièrement préféré.

Selon l'invention, le ou les sels de manganèse sont de préférence présents à une concentration comprise entre 0,002 et 5 % en poids environ d'équivalents métal par rapport au poids total de la composition tinctoriale. Encore plus préférentiellement, cette concentration est comprise entre 0,005 et 0,5 % en poids environ d'équivalents métal par rapport au poids total de la composition tinctoriale.

Les ortho-diamino pyrazoles conformes à l'invention et / ou le ou leurs sels d'addition avec un acide représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement supérieur ou égal à 7, et de préférence compris entre 9 et 12 environ. Il peut être ajusté à la valeur désirée au moyen d'agents alcalinisants ou éventuellement acidifiants habituellement utilisés en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (II) suivante : 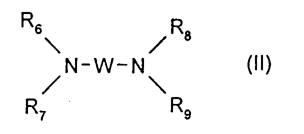 dans laquelle W est un reste propylène éventuellement substitué par un groupement hydroxyle ou un radical alkyle en C₁-C₄ ; R₆, R₇, R₈ et R₉, identiques ou différents, représentent un atome d'hydrogène, un radical alkyle en C₁-C₄ ou hydroxyalkyle en C₁-C₄.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

La composition tinctoriale conforme à l'invention peut également renfermer au moins un coupleur et/ou au moins un colorant direct, notamment pour modifier les nuances ou les enrichir en reflets.

Les coupleurs utilisables dans la composition tinctoriale mise en oeuvre dans le procédé conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, et leurs sels d'addition avec un acide.

Ces coupleurs peuvent notamment être choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

Les sels d'addition avec un acide du ou des ortho-diamino pyrazoles conformes à l'invention et/ou des coupleurs utilisables dans la composition tinctoriale conforme à l'invention sont notamment choisis parmi les chlorhydrates, les bromhydrates, les sulfates et les tartrates, les lactates et les acétates.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir le ou les éventuels composés complémentaires mentionnés ci-avant, de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, de shampooings ou sous toute autre forme appropriée pour réaliser une teinture des fibres kératiniques, et notamment des cheveux humains.

Les compositions tinctoriales sont de préférence préparées sous gaz inerte tel que l'argon et conditionnées à l'abri de l'air de façon à éviter toute oxydation prématurée des ortho-diamino pyrazoles conformes à l'invention.

Un autre objet de l'invention est un dispositif à plusieurs compartiments ou "kit" de teinture ou tout autre système de conditionnement à plusieurs compartiments dont un premier compartiment renferme une composition A contenant, dans un milieu approprié pour la teinture, au moins un ortho-diamino pyrazole incapable d'être révélé efficacement par le seul oxygène de l'air et/ou au moins l'un de ses sels d'addition avec un acide et un second compartiment renferme une composition B contenant, dans un milieu approprié pour la teinture, au moins un sel de manganèse tel que défini précédemment. Ces dispositifs peuvent être équipés d'un moyen permettant de délivrer sur les cheveux le mélange souhaité, tel que les dispositifs décrits dans le brevet FR-2 586 913 au nom de la demanderesse.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, pendant un temps suffisant pour développer la coloration désirée, la révélation de la coloration étant effectuée à l'air et de préférence sans autre agent oxydant que l'oxygène de l'air.

Selon une forme de réalisation préférée du procédé de teinture conforme à l'invention, la composition tinctoriale est appliquée sur les fibres kératiniques avec un temps de pose variant de préférence entre 30 secondes et 40 minutes, après quoi les fibres sont rincées, éventuellement lavées au shampooing puis séchées.

Lorsque la composition tinctoriale conforme à l'invention se présente sous la forme d'un kit de teinture à plusieurs compartiments, le procédé conforme à l'invention comporte alors une étape préliminaire consistant à mélanger, au moment de l'emploi, les composants A et B définis ci-dessus, le mélange obtenu étant ensuite appliqué sur les fibres kératiniques selon les conditions définies précédemment.

Les exemples qui suivent sont destinés à illustrer l'invention sans pour autant en limiter la portée.

### EXEMPLES

### EXEMPLE 1 COMPARATIF

On a préparé les compositions tinctoriales 1A, 1B, 1C et 1D suivantes :

| Composition 1A ne faisant pas partie de l'invention : | |
|---|---|
| - Dichlorhydrate de 4,5-diamino pyrazole | 0,382 g |
| - Dichlorhydrate de 2,4-diamino 1-(β-hydroxyéthyloxy) benzène | 0,482 g |
| - Alcool éthylique | 10 g |
| - Monoéthanolamine | 5 g |
| - Eau déminéralisée q.s.p. | 100 g |
| pH = 10,5 | |

| Composition 1B faisant partie de l'invention : | |
|---|---|
| - Dichlorhydrate de 4,5-diamino pyrazole | 0,382 g |
| - Dichlorhydrate de 2,4-diamino 1-(β-hydroxyéthyloxy) benzène | 0,482 g |
| - **Diacétate de manganèse, tétrahydrate** | **0,1 g** |
| - Alcool éthylique | 10 g |
| - Monoéthanolamine | 5 g |
| - Eau déminéralisée q.s.p. | 100 g |
| pH = 10,5 | |

| Composition 1C ne faisant pas partie de l'invention : | |
|---|---|
| - **Ortho-diamino benzène** | **0,218 g** |
| - Dichlorhydrate de 2,4-diamino 1-(β-hydroxyéthyloxy) benzène | 0,482 g |
| - Alcool éthylique | 10 g |
| - Monoéthanolamine | 5 g |
| - Eau déminéralisée q.s.p. | 100 g |
| pH = 10,5 | |

| Composition 1D ne faisant pas partie de l'invention : | |
|---|---|
| - **Ortho-diamino benzène** | **0,218 g** |
| - Dichlorhydrate de 2,4-diamino 1-(β-hydroxyéthyloxy) benzène | 0,482 g |
| - **Diacétate de manganèse, tétrahydrate** | **0,1 g** |
| - Alcool éthylique | 10 g |
| - Monoéthanolamine | 5 g |
| - Eau déminéralisée q.s.p. | 100 g |
| pH = 10,5 | |

Les compositions 1A, 1B, 1C et 1D ont été préparées sous argon.

Chaque composition 1A, 1B, 1C et 1D a été appliquée sur des mèches de cheveux secs gris naturels à 90 % de blancs pendant 30 minutes. Les mèches de cheveux ont ensuite été rincées, lavées avec un shampooing standard puis séchées.

La mèche ayant subi l'application de la composition 1A ne faisant pas partie de l'invention (car ne contenant pas de sel de manganèse) ne présentait aucune coloration alors que la mèche ayant subi l'application de la composition 1B conforme à l'invention (car contenant un sel de manganèse) a été teinte dans une nuance violine intense.

D'autre part les mèches ayant subi l'application des compositions 1C ou 1D ne faisant pas partie de l'invention car contenant de l'ortho-diamino benzène et non un ortho-diamino pyrazole conforme à l'invention, n'ont conduit à aucune coloration, en présence ou en absence de sel de manganèse.

### EXEMPLE 2

On a préparé, sous argon, un shampooing, conforme à l'invention, de composition suivante :

| | |
|---|---|
| - Dichlorhydrate de 4,5-diamino pyrazole | 0,382 g |
| - Dichlorhydrate de 2,4-diamino 1-(β-hydroxyéthyloxy) benzène | 0,482 g |
| - **Diacétate de manganèse, tétrahydrate** | **0,1 g** |
| - Laurylsulfate de sodium | 1,5 g |
| - Monoéthanolamine | 3 g |
| - Eau déminéralisée q.s.p. | 100 g |
| pH = 10 | |

Ce shampooing a été appliqué et émulsionné pendant 2 minutes sur des cheveux humides gris naturels à 90 % de blancs. Après rinçage et séchage, les cheveux présentaient une couleur rose.

### EXEMPLE 3

On a préparé, sous argon, la composition tinctoriale conforme à l'invention suivante :

| | |
|---|---|
| - Dichlorhydrate de 4,5-diamino pyrazole | 0,382 g |
| - 4-hydroxyindole | 0,266 g |
| - **Diacétate de manganèse, tétrahydrate** | **0,1 g** |
| - Alcool éthylique | 10 g |
| - Monoéthanolamine | 5 g |
| - Eau déminéralisée q.s.p. | 100 g |
| pH = 10,5 | |

Cette composition a été appliquée sur des cheveux gris permanentés à 90 % de blancs pendant 30 minutes. Les cheveux ont ensuite été rincés, lavés au shampooing puis séchés.

Les mèches de cheveux ont été teintes dans une nuance violet intense.

## Revendications

1. Composition pour la teinture des fibres kératiniques et en particulier des fibres kératiniques humaines telles que les cheveux, caractérisée par le fait qu'elle comprend, dans un milieu approprié pour la teinture :
- au moins un ortho-diamino pyrazole et / ou au moins l'un de ses sels d'addition avec un acide, incapable d'être révélé efficacement par le seul oxygène de l'air,
- et au moins un sel de manganèse.

2. Composition selon la revendication 1, caractérisée par le fait que les ortho-diamino pyrazoles sont choisis parmi les composés de formule (I) suivante, et leurs sels d'addition avec un acide : 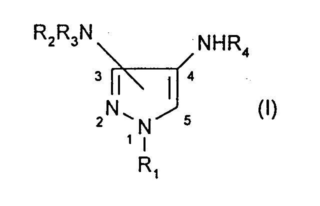 dans laquelle :
- R₁, R₂ et R₄, identiques ou différents, représentent un atome d'hydrogène, un radical alkyle en C₁-C₄, monohydroxyalkyle en C₂-C₄, benzyle ou phényle,
- R₃ représente un atome d'hydrogène, un radical alkyle en C₁-C₄ ou monohydroxyalkyle en C₂-C₄,
le groupement amino NR₂R₃ occupant soit la position 3, soit la position 5.

3. Composition selon la revendication 2, caractérisée par le fait que les ortho-diamino pyrazoles sont choisis parmi le 4,5-diamino pyrazole, le 4,5-diamino 1-méthyl pyrazole, le 1-benzyl 4,5-diamino pyrazole, le 3,4-diamino pyrazole, et leurs sels d'addition avec un acide.

4. Composition selon la revendication 3, caractérisée par le fait qu'elle contient du 4,5-diamino pyrazole et / ou au moins l'un de ses sels d'addition avec un acide.

5. Composition selon l'une quelconque des revendications précédentes, caractérisée par le fait que le manganèse présente un degré d'oxydation égal à 2 ou à 3.

6. Composition selon l'une quelconque des revendications précédentes, caractérisée par le fait que le ou les sels de manganèse sont choisis parmi le diacétate de manganèse tétrahydrate, le dichlorure de manganèse et ses hydrates, les sulfates de manganèse, les carbonates de manganèse, les dihydrogénocarbonates de manganèse, l'acétylacétonate de manganèse, le triacétate de manganèse et ses hydrates et le trichlorure de manganèse.

7. Composition selon l'une quelconque des revendications précédentes, caractérisée par le fait que le ou les sels de manganèse sont présents à une concentration comprise entre 0,002 et 5 % en poids d'équivalents métal par rapport au poids total de la composition tinctoriale.

8. Composition selon la revendication 7, caractérisée par le fait que le ou les sels de manganèse sont présents à une concentration comprise entre 0,005 et 0,5 % en poids d'équivalents métal par rapport au poids total de la composition tinctoriale.

9. Composition selon l'une quelconque des revendications précédentes, caractérisée par le fait que le ou les ortho-diamino pyrazoles et / ou le ou leurs sels d'addition avec un acide représentent de préférence de 0,0005 à 12 % en poids du poids total de la composition tinctoriale.

10. Composition selon la revendication 9, caractérisée par le fait que le ou les ortho-diamino pyrazoles et / ou le ou leurs sels d'addition avec un acide représentent de préférence de 0,005 à 6 % en poids du poids total de la composition tinctoriale.

11. Composition selon l'une quelconque des revendications précédentes, caractérisée par le fait que le milieu approprié pour la teinture est généralement constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

12. Composition selon l'une quelconque des revendications précédentes, caractérisée par le fait qu'elle présente un pH supérieur ou égal à 7.

13. Composition selon la revendication 12, caractérisée par le fait qu'elle présente un pH compris entre 9 et 12.

14. Composition selon l'une quelconque des revendications précédentes, caractérisée par le fait qu'elle renferme en outre au moins un coupleur et/ou au moins un colorant direct.

15. Composition selon la revendication 14, caractérisée par le fait que les coupleurs sont choisis parmi métaphénylènediamines, les méta-aminophénols, les métadiphénols et les coupleurs hétérocycliques tels que par exemple les dérivés indoliques, les dérivés indoliniques, et leurs sels d'addition avec un acide.

16. Composition selon l'une quelconque des revendications précédentes, caractérisée par le fait que lesdits sels d'addition avec un acide du ou des ortho-diamino pyrazoles et/ou des coupleurs sont choisis parmi les chlorhydrates, les bromhydrates, les sulfates et les tartrates, les lactates et les acétates.

17. Procédé de teinture des fibres kératiniques et en particulier des fibres kératiniques humaines telles que les cheveux, caractérisé par le fait qu'on applique sur ces fibres une composition tinctoriale telle définie à l'une quelconque des revendications 1 à 16, pendant un temps suffisant pour développer la coloration désirée, la révélation de la coloration étant effectuée à l'air sans autre agent oxydant que l'oxygène de l'air.

18. Procédé selon la revendication 17, caractérisé par le fait que la composition tinctoriale est appliquée sur les fibres kératiniques avec une temps de pose variant entre 30 secondes et 40 minutes.

19. Dispositif à plusieurs compartiments ou "kit" de teinture à plusieurs compartiments, caractérisé par le fait qu'un premier compartiment renferme une composition A contenant, dans un milieu approprié pour la teinture, au moins un ortho-diamino pyrazole incapable d'être révélé efficacement par le seul oxygène de l'air et/ou au moins l'un de ses sels d'addition avec un acide et un second compartiment renferme une composition B contenant, dans un milieu approprié pour la teinture, au moins un sel de manganèse tel que défini à l'une quelconque des revendications 5 ou 6.

## Claims

1. Composition for dyeing keratinous fibres and in particular human keratinous fibres such as the hair, characterized in that it comprises, in an appropriate medium for dyeing:
- at least one ortho-diaminopyrazole and/or at least one of its addition salts with an acid, which is incapable of being effectively developed with atmospheric oxygen alone,
- and at least one manganese salt.

2. Composition according to Claim 1, characterized in that the ortho-diaminopyrazoles are chosen from the compounds of the following formula (I), and their addition salts with an acid: 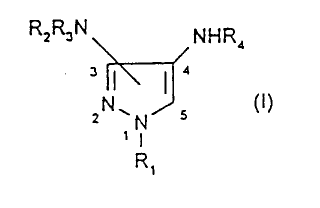 in which:
- R₁, R₂ and R₄, which are identical or different, represent a hydrogen atom or a C₁-C₄ alkyl, C₂-C₄ monohydroxyalkyl, benzyl or phenyl radical,
- R₃ represents a hydrogen atom or a C₁-C₄ alkyl or C₂-C₄ monohydroxyalkyl radical,
the amino group NR₂R₃ occupying either the 3-position or the 5-position.

3. Composition according to Claim 2, characterized in that the ortho-diaminopyrazoles are chosen from 4,5-diaminopyrazole, 4,5-diamino-1-methylpyrazole, 1-benzyl-4,5-diaminopyrazole, 3,4-diaminopyrazole, and their addition salts with an acid.

4. Composition according to Claim 3, characterized in that it contains 4,5-diaminopyrazole and/or at least one of its addition salts with an acid.

5. Composition according to any one of the preceding claims, characterized in that the manganese has an oxidation number equal to 2 or 3.

6. Composition according to any one of the preceding claims, characterized in that the manganese salt or salts are chosen from manganese diacetate tetrahydrate, manganese dichloride and its hydrates, manganese sulphates, manganese carbonates, manganese dihydrogen carbonates, manganese acetylacetonate, manganese triacetate and its hydrates and manganese trichloride.

7. Composition according to any one of the preceding claims, characterized in that the manganese salt or salts are present at a concentration of between 0.002 and 5 % of metal equivalent weight relative to the total weight of the dyeing composition.

8. Composition according to Claim 7, characterized in that the manganese salt or salts are present at a concentration of between 0.005 and 0.5 % of metal equivalent weight relative to the total weight of the dyeing composition.

9. Composition according to any one of the preceding claims, characterized in that the ortho-diamino-pyrazole(s) and/or the or their addition salts with an acid preferably represent 0.0005 to 12 % by weight of the total weight of the dyeing composition.

10. Composition according to Claim 9, characterized in that the ortho-diaminopyrazole(s) and/or the or their addition salts with an acid preferably represent 0.005 to 6 % by weight of the total weight of the dyeing composition.

11. Composition according to any one of the preceding claims, characterized in that the appropriate medium for dyeing generally consists of water or a mixture of water and at least one organic solvent chosen from C₁-C₄ lower alkanols, glycerol, glycols and glycol ethers, aromatic alcohols, analogous products and mixtures thereof.

12. Composition according to any one of the preceding claims, characterized in that it has a pH greater than or equal to 7.

13. Composition according to Claim 12, characterized in that it has a pH of between 9 and 12.

14. Composition according to any one of the preceding claims, characterized in that it contains, in addition, at least one coupler and/or at least one direct dye.

15. Composition according to Claim 14, characterized in that the couplers are chosen from meta-phenylenediamines, meta-aminophenols, meta-diphenols and heterocyclic couplers such as for example indole derivatives, indoline derivatives, and their addition salts with an acid.

16. Composition according to any one of the preceding claims, characterized in that the said addition salts with an acid of the ortho-diaminopyrazole(s) and/or of the couplers are chosen from hydrochlorides, hydrobromides, sulphates and tartrates, lactates and acetates.

17. Process for dyeing keratinous fibres and in particular human keratinous fibres such as the hair, characterized in that a dyeing composition as defined in any one of Claims 1 to 16 is applied to these fibres for a time sufficient to develop the desired colour, the colour being developed in the air without any oxidizing agent other than atmospheric oxygen.

18. Process according to Claim 17, characterized in that the dyeing composition is applied to the keratinous fibres with an exposure time varying between 30 seconds and 40 minutes.

19. Multicompartment device or multicompartment dyeing "kit", characterized in that a first compartment comprises a composition A containing, in an appropriate medium for dyeing, at least one ortho-diaminopyrazole incapable of being effectively developed with atmospheric oxygen alone and/or at least one of its addition salts with an acid and a second compartment comprises a composition B containing, in an appropriate medium for dyeing, at least one manganese salt as defined in either of Claims 5 and 6.

## Patentansprüche

1. Zusammensetzung für die Färbung keratinischer Fasern und insbesonders menschlicher keratinischer Fasern, wie z.B. Haare, dadurch gekennzeichnet, daß sie in einem für die Färbung geeigneten Medium enthält:
- mindestens ein ortho-Diaminopyrazol und/oder mindestens eines seiner Additionssalze mit einer Säure, das allein mit Luftsauerstoff nicht wirksam entwickelt werden kann,
- und mindestens ein Mangansalz.

2. Zusammensetzung nach Anspruch 1, dadurch gekennzeichnet, daß die o-Diaminopyrazole ausgewählt sind unter den Verbindungen der folgenden Formel (I) und deren Additionssalzen mit einer Säure 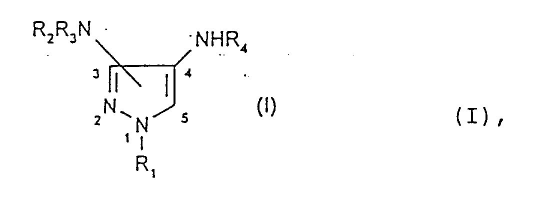 in der bedeuten:
- R₁, R₂ und R₄, die gleich oder verschieden sind, ein Wasserstoffatom, eine C₁₋₄-Alkylgruppe, eine C₂₋₄-Monohydroxyalkylgruppe, Benzyl oder Phenyl,
- R₃ ein Wasserstoffatom, eine C₁₋₄-Alkylgruppe oder eine C₂₋₄-Monohydroxyalkylgruppe,
wobei die Aminogruppe NR₂R₃ entweder in 3-Stellung oder 5-Stellung angeordnet ist.

3. Zusammensetzung nach Anspruch 2, dadurch gekennzeichnet, daß die o-Diaminopyrazole unter 4,5-Diaminopyrazol, 4,5-Diamino-1-methylpyrazol, 1-Benzyl-4,5-diaminopyrazol, 3,4-Diaminopyrazol und deren Additionssalzen mit einer Säure ausgewählt sind.

4. Zusammensetzung nach Anspruch 3, dadurch gekennzeichnet, daß sie 4,5-Diaminopyrazol und/oder mindestens eines seiner Additionssalze mit einer Säure enthält.

5. Zusammensetzung nach einem der vorhergehenden Ansprüche, dadurch gekennzeichnet, daß das Mangan die Oxidationsstufe 2 oder 3 aufweist.

6. Zusammensetzung nach einem der vorhergehenden Ansprüche, dadurch gekennzeichnet, daß das oder die Mangansalze unter Mangandiacetat-Tetrahydrat, Mangandichlorid und seinen Hydraten, Mangansulfaten, Mangancarbonaten, Mangandihydrogencarbonaten, Manganacetylacetonat, Mangantriacetat und seinen Hydraten und Mangantrichlorid ausgewählt sind.

7. Zusammensetzung nach einem der vorhergehenden Ansprüche, dadurch gekennzeichnet, daß das oder die Mangansalze in einer Konzentration von 0,002 bis 5 Gew.-% an Metalläquivalenten, bezogen auf das Gesamtgewicht der Färbezusammensetzung, enthalten sind.

8. Zusammensetzung nach Anspruch 7, dadurch gekennzeichnet, daß das oder die Mangansalze in einer Konzentration von 0,005 bis 0,5 Gew.-% an Metalläquivalenten, bezogen auf das Gesamtgewicht der Färbezusammensetzung, enthalten sind.

9. Zusammensetzung nach einem der vorhergehenden Ansprüche, dadurch gekennzeichnet, daß das oder die o-Diaminopyrazole und/oder dessen oder deren Additionssalze mit einer Säure vorzugsweise 0,0005 bis 12 Gew.-% des Gesamtgewicht der Färbezusammensetzung ausmachen.

10. Zusammensetzung nach Anspruch 9, dadurch gekennzeichnet, daß das oder die o-Diaminopyrazole und/oder dessen oder deren Additionssalze mit einer Säure vorzugsweise 0,005 bis 6 Gew.-% des Gesamtgewicht der Färbezusammensetzung ausmachen.

11. Zusammensetzung nach einem der vorhergehenden Ansprüche, dadurch gekennzeichnet, daß das für die Färbung geeignete Medium im allgemeinen aus Wasser oder aus einem Gemisch aus Wasser und mindestens einem organischem Lösungsmittel besteht, das unter niederen Alkanolen mit 1 bis 4 Kohlenstoffatomen, Glycerin, Glykolen und Glykolethern, aromatischen Alkoholen, entsprechenden Produkten und deren Gemischen ausgewählt ist.

12. Zusammensetzung nach einem der vorhergehenden Ansprüche, dadurch gekennzeichnet, daß sie einen pH-Wert von 7 oder darüber aufweist.

13. Zusammensetzung nach Anspruch 12, dadurch gekennzeichnet, daß sie einen pH-Wert von 9 bis 12 aufweist.

14. Zusammensetzung nach einem der vorhergehenden Ansprüche, dadurch gekennzeichnet, daß sie außerdem mindestens einen Kuppler und/oder mindestens ein direktziehendes Färbemittel enthält.

15. Zusammensetzung nach Anspruch 14, dadurch gekennzeichnet, daß die Kuppler unter m-Phenylendiaminen, m-Aminophenolen, m-Diphenolen und heterocyclischen Kupplern, wie z.B. Indolderivaten, Indolinderivaten und deren Additionssalzen mit einer Säure ausgewählt sind.

16. Zusammensetzung nach einem der vorhergehenden Ansprüche, dadurch gekennzeichnet, daß die obengenannten Additionssalze mit einer Säure des oder der o-Diaminopyrazole und/oder der Kuppler unter Hydrochloriden, Hydrobromiden, Sulfaten und Tartaten, Lactaten und Acetaten ausgewählt sind.

17. Verfahren zur Färbung keratinischer Fasern und insbesondere menschlicher keratinischer Fasern wie Haaren, dadurch gekennzeichnet, daß auf diese Fasern eine wie in einem der Ansprüche 1 bis 16 definierte Färbezusammensetzung während einer Zeit, die für die Entwicklung der gewünschten Farbe ausreicht, angewendet wird, wobei die Entwicklung der Farbe mit Luft und Luftsauerstoff als einzigem Oxidationsmittel erfolgt.

18. Verfahren nach Anspruch 17, dadurch gekennzeichnet, daß die Färbezusammensetzung während einer Ruhezeit von 30 s bis 40 min auf die keratinischen Fasern angewendet wird.

19. Vorrichtung mit mehreren Abteilungen oder Färbe-Kit mit mehreren Abteilungen, dadurch gekennzeichnet, daß eine erste Abteilung eine Zusammensetzung A enthält, die in einem für die Färbung geeigneten Medium mindestens ein o-Diaminopyrazol, das nicht wirksam allein mit Luftsauerstoff der Luft entwickelt werden kann, und/oder mindestens eines seiner Additionssalze mit einer Säure, enthält, und daß eine zweite Abteilung eine Zusammensetzung B enthält, die in einem für die Färbung geeigneten Medium mindestens ein wie in einem der Ansprüche 5 und 6 definiertes Mangansalz enthält.
